# EUROPEAN PATENT APPLICATION

(11) **EP 1 457 210 A1**
(43) Date of publication of application: **15.09.2004**
(21) Application number: 02805029.2
(22) Date of filing: 19.12.2002
(51) Int. Cl.: A61K 38/17, A61K 38/00, A61P 25/00, A61P 25/14, A61P 25/28, A61P 43/00

(54) **REMEDIES AND/OR PREVENTIVES FOR CONFORMATIONAL DISEASES**

(30) Priority: 19.12.2001 JP 2001386699
(71) Applicant: Itoham Foods Inc., Kobe-shi, Hyogo 657-0037 (JP)
(72) Inventor: ONOUE, Satomi; c/o ITOHAM FOODS INC., Moriya-shi, Ibaraki302-0104 (JP); KASHIMOTO, Kazuhisa; c/o ITOHAM FOODS INC., Moriya-shi, Ibaraki302-0104 (JP)
(74) Representative: Woods, Geoffrey Corlett
(86) International application number: PCT/JP2002/013311
(87) International publication number: WO 2003/051387

(57) **Abstract**

The present invention provides a therapeutic and/or prophylactic agent against conformational diseases. An object of the present invention is to provide a therapeutic and/or prophylactic agent against conformational diseases, which comprise, as an active ingredient, a neuropeptide or a pharmacologically acceptable salt thereof having neuroprotective action.

## Description

### TECHNICAL FIELD

The present invention relates to a therapeutic and/or prophylactic agent against conformational diseases, which contains as an active ingredient a neuropeptide or a pharmacologically acceptable salt thereof having a neuroprotective action, and relates to a pharmaceutical composition which comprises the agent.

### BACKGROUND ART

Based on the hypothesis proposed by Harris that the adenohypophysis is controlled by the humoral factor which is produced in the hypothalamus and then transported via the portal vein to the anterior lobe of the hypophysis, hypothalamic hormones such as thyrotropin-releasing hormone (TRH), luteinizing hormone-releasing hormone (LH-RH), growth hormone release-inhibiting hormone (somatostatin), growth hormone (GRH)-releasing factor, corticotropin (CRH)-releasing factor have been identified over the past 30 years. These substances are neuropeptides acting as nerve transmitters and neurological function-controlling factors. After being released from the hypothalamus, the neuropeptides act on the hypophysis to promote and suppress the hormone secreted by various stimuli. However, whether the above-identified substances are the all neuropeptides is a matter of argument. Arimura and colleagues have modified the conventional strategy for isolating and identifying neuropeptides. Specifically, they used for isolation the activation of the intracellular information transmission systems (particularly, the cAMP system that is important for the secretion of CRH, GH by GRH and ACTH) as an indicator in the cells of anterior lobe of hypophysis, attempted to isolate a new neuropeptide from the hypothalamus, and finally identified the neuropeptide (Miyata A et al, Biochem Biophys Res Commun (1989), 164, 567-574). Since this peptide was isolated using the activation of adenylate cyclase of the hypophysis as an indicator, it was named Pituitary Adenylate Cyclase Activating Polypeptide (hereinafter referred to as PACAP).

Further, there is the Vasoactive Intestinal Peptide (hereinafter, referred to as VIP) as a neuropeptide that has been found in nature earlier than PACAP. VIP is a type of neuropeptide that is secreted from the intestine, and has an action to relax the smooth muscles of the digestive tract. Further, the structure of 27 amino acids from the N-terminal of PACAP is extremely analogous to that of VIP. VIP and PACAP have a lot in common with secretin, glucagon and the like in terms of peptide length and amino acid sequence, and amidated C-terminus, so that they generally belong to the glucagon-secretin family.

In the mean time, there have been many diseases in the nervous system having unknown mechanisms. Particularly, there have been many unidentified aspects of the pathological conditions of neurodegenerative diseases. At present, neurodegenerative disease is defined as "a group of diseases that develops various nervous/mental symptoms as a result of progressive degeneration and deciduation of a type of neuron group, which is specific for each disease, due to metabolic errors with unknown causes." Neurodegenerative disease includes incredibly various diseases such as Alzheimer disease and Parkinson disease whose frequency is extremely high and most of them is sporadic, and polyglutamine disease that is relatively rare as an individual disease but varies in disease type and all the disease types show hereditary characteristics. However, a prospect that is being rapidly brightened recently is about the mechanism of neurodegenerative disease that abnormality occurs in the folding of specific proteins, which is a causative gene product of the disease so as to form insoluble accumulations, which mainly form a β sheet structure, and new abnormal functions are acquired during this process, so that cell death may result. As a result, the concept of conformational disease focusing on a specific protein that is generated by changes in the higher-order structure of protein is starting to be rapidly penetrated. A characteristic of these conformational diseases is that a specific protein, which is accumulated within the brain, is thought to be essential for the onset. For example, causative substances that are currently proposed are prion protein for Creutzfeldt-Jakob disease, mad cow disease (cattle spongy encephalopathy) and Gerstmann-Straussler syndrome; polyglutamine for bulbar spinal muscular atrophy, Huntington's disease, spinocerebellar ataxia, Dentatorubral Pallidoluysian atrophy; α-Synuclein for Parkinson disease, Lewy Body Dementia and polyphyletic atrophy; β-amyloid for Alzheimer disease and Down's syndrome; SOD1 for familial amyotrophic lateral sclerosis; Tau for FTDP-17, progressive supranuclear palsy, cortical-basal ganglia degeneration and Pick's disease; Abri for familial British dementia; and neuroserpin for familial dementia [Cell Technology (2001) 20, 1475].

The causative substances and the action mechanisms of these conformational diseases are now being gradually elucidated. The prompt development of therapeutically and/or prophylactically effective pharmaceutical products is now expected. However, there have been few reports so far on pharmaceutical products that are efficient and effective against these conformational diseases, and there have been no report regarding any therapeutic and/or prophylactic pharmaceutical product against conformational diseases using the above neuropeptide.

### DISCLOSURE OF THE INVENTION

Therefore, an object of the present invention is to provide a therapeutic and/or prophylactic agent against conformational diseases, which contains as an active ingredient, a neuropeptide or a pharmacologically acceptable salt thereof having a neuroprotective action, and a pharmaceutical composition comprising the agent.

As a result of intensive studies to achieve the above objective, we have completed the present invention by finding a neuropeptide that has a neuroprotective action against a specific protein that can cause the onset of a conformational disease.

The present invention provides the following (1) to (7):
(1) A therapeutic and/or prophylactic agent against conformational diseases, which contains, as an active ingredient, a neuropeptide having a neuroprotective action or a pharmacologically acceptable salt thereof.
(2) The agent of (1), wherein the neuropeptide comprises at least 23 residues from the N terminus of a peptide that is represented by the following formula (I): 
His-Ser-Asp-A-B-Phe-Thr-Asp-C-Tyr-D-Arg-E-Arg-F-Gln-G-Ala-Val-I-J-Tyr-Leu
-K-L-M-Leu-N (I)
 (wherein A represents Ala or Gly; B represents Ile or Val; C represents Asn or Ser; D represents Thr or Ser; E represents Leu or Tyr; F and J respectively represent Lys or Arg; I represents Lys, Arg or Gln; but at least one of F, I and J represents Arg; G represents Met, Leu or nLeu; K represents Asn or Ala; L represents Ser or Ala; M represents Ile or Val; and N represents -NH₂ or Asn-NH₂).
(3) The agent of (1), wherein the neuropeptide is represented by the following formula (II): 
His-Ser-Asp-A-B-Phe-Thr-Asp-C-Tyr-D-Arg-E-Arg-F-Gln-G-Ala-Val-I-J-Tyr-Leu
-K-L-M-Leu-P-Gly-Q-R (II)
 (wherein A represents Ala or Gly; B represents Ile or Val; C represents Asn or Ser; D represents Thr or Ser; E represents Leu or Tyr; F and J respectively represent Lys or Arg; I represents Lys, Arg or Gln; but at least one of F, I and J represents Arg; G represents Met, Leu or nLeu; K represents Asn or Ala; L represents Ser or Ala; M represents Ile or Val; P represents Asn or a chemical bond; Q represents Lys, Arg, Lys-Arg, Arg-Arg or a chemical bond; and R represents -OH or -NH₂).
(4) The agent of (1), wherein the neuropeptide is represented by the following formula (III): 
His-Ser-Asp-A-B-Phe-Thr-Asp-C-Tyr-D-Arg-E-Arg-F-Gln-G-Ala-Val-I-J-Tyr-Leu
-K-L-M-Leu-N-Gly-O-P-Tyr-Q-Gln-R-Val-S-Asn-T-U (III)
 (wherein A represents Ala or Gly; B represents Ile or Val; C represents Asn or Ser; D represents Thr or Ser; E represents Leu or Tyr; F, J, O, P, Q, R, S and T respectively represent Lys or Arg; I represents Lys, Arg or Gln; but at least one of F, I and J represents Arg; G represents Met, Leu or nLeu; K represents Asn or Ala; L represents Ser or Ala; M represents Ile or Val; N represents a chemical bond or Asn; and U represents -OH or -NH₂).
(5) The agent of any one of (1) to (4), wherein the conformational disease is developed by the accumulation of prion protein, polyglutamic acid, α-Synuclein, β-amyloid, SOD1, Tau, Abri or neuroserpin.
(6) The agent of any one of (1) to (5), wherein the conformational disease is Creutzfeldt-Jakob disease, mad cow disease (cattle spongy encephalopathy), Gerstmann-Straussler syndrome, bulbar spinal muscular atrophy, Huntington's disease, spinocerebellar ataxia, Dentatorubral Pallidoluysian atrophy, Parkinson disease, Lewy Body Dementia, polyphyletic atrophy, Alzheimer disease, Down's syndrome, familial amyotrophic lateral sclerosis, FTDP-17, progressive supranuclear palsy, cortical-basal ganglia degeneration, Pick's disease, familial British dementia or familial dementia.
(7) A pharmaceutical composition, comprising the agent of any one of (1) to (6).

The present specification includes part or all of the contents as disclosed in the specification and/or the drawings of Japanese Patent Application No. 2001-386699, which is a priority document of the present application.

### BRIEF DESCRIPTION OF DRAWINGS

Figure 1 shows the effect of abnormal prion on cell death.
Figure 2 shows the degree of the neuroprotective action of the neuropeptide against abnormal prion. "•" indicates PACAP, and "▲" indicates VIP.
Figure 3 shows the effect of β-amyloid on cell death. "□" indicates 5% horse serum and 5% newborn calf serum-containing DMEM, shaded "□" indicates 0.25% horse serum and 0.25% newborn calf serum-containing DMEM, and "■" indicates 0.2 µM insulin-containing serum free DMEM.
Figure 4 shows the degree of the neuroprotective action of the neuropeptide against β-amyloid. "□" indicates control, "■" indicates β-amyloid 10⁻⁵M, "•" indicates PACAP27 10⁻⁹M, "#" indicates P<0.05 and "##" indicates P<0.01.
Figure 5 shows the neuroprotective actions of the neuropeptides PACAP27, VIP and humanin, and a cell-permeable cAMP derivative. "#" indicates P<0.05, "##" indicates P<0.01 (for control), "*" indicates P<0.05 and "**" indicates P<0.01 (for β-amyloid-treated group).
Figure 6 shows the relationship between the concentrations of neuropeptides and the neuroprotective action. "•" indicates PACAP and "▲" indicates VIP.
Figure 7 shows the caspase-3 activating action of β-amyloid and the effect of the neuropeptide to suppress the action. "#" indicates P<0.01 (for control), "*" indicates P<0.05 and "**" indicates P<0.01 (for β-amyloid-treated group).

### Description of Sequence Listing

SEQ ID NO: 1: Xaa at position 4represents Ala or Gly.
SEQ ID NO: 1: Xaa at position 5 represents Ile or Val.
SEQ ID NO: 1: Xaa at position 9 represents Asn or Ser.
SEQ ID NO: 1: Xaa at position 11 represents Thr or Ser.
SEQ ID NO: 1: Xaa at position 13 represents Leu or Tyr.
SEQ ID NO: 1: Xaa at position 15 represents Lys or Arg.
SEQ ID NO: 1: Xaa at position 17 represents Met or Leu or nLeu.
SEQ ID NO: 1: Xaa at position 20 represents Lys or Arg or Gln.
SEQ ID NO: 1: Xaa at position 21 represents Lys or Arg.
SEQ ID NO: 1: Xaa at position 24 represents Asn or Ala.
SEQ ID NO: 1: Xaa at position 25 represents Ser or Ala.
SEQ ID NO: 1: Xaa at position 26 represents Ile or Val.
SEQ ID NO: 10: Xaa at position 2 represents Lys or Arg.
SEQ ID NO: 10: Xaa at position 3 represents Lys or Arg.
SEQ ID NO: 10: Xaa at position 5 represents Lys or Arg.
SEQ ID NO: 10: Xaa at position 7 represents Lys or Arg.
SEQ ID NO: 10: Xaa at position 9 represents Lys or Arg.
SEQ ID NO: 10: Xaa at position 11 represents Lys or Arg.
SEQ ID NO: 11: Xaa at position 3 represents Lys or Arg.
SEQ ID NO: 11: Xaa at position 4 represents Lys or Arg.
SEQ ID NO: 11: Xaa at position 6 represents Lys or Arg.
SEQ ID NO: 11: Xaa at position 8 represents Lys or Arg.
SEQ ID NO: 11: Xaa at position 10 represents Lys or Arg.
SEQ ID NO: 11: Xaa at position 12 represents Lys or Arg.

### BEST MODE FOR CARRYING OUT THE INVENTION

The present invention will be described in detail as follows.

The neuropeptide of the present invention, which has a neuroprotective action, is specifically a peptide comprising at least 23 residues from the N-terminus of the peptide comprising the amino acid sequence of SEQ ID NO: 1, or a peptide comprising an amino acid sequence wherein Asn or an amino acid sequence of any one of SEQ ID NOS: 2 to 12 is added to the C-terminus of the peptide comprising the amino acid sequence of SEQ ID NO: 1.

His-Ser-Asp-Xaa-Xaa-Phe-Thr-Asp-Xaa-Tyr-Xaa-Arg-Xaa-Arg-Xaa-Gln-Xaa-Ala
-Val-Xaa-Xaa-Tyr-Leu-Xaa-Xaa-Xaa-Leu (SEQ ID NO: 1)
Gly-Lys (SEQ ID NO: 2)
Gly-Arg (SEQ ID NO: 3)

Gly-Lys-Arg (SEQ ID NO: 4)
Gly-Arg-Arg (SEQ ID NO: 5)
Asn-Gly-Lys (SEQ ID NO: 6)
Asn-Gly-Arg (SEQ ID NO: 7)
Asn-Gly-Lys-Arg (SEQ ID NO: 8)
Asn-Gly-Arg-Arg (SEQ ID NO: 9)
Gly-Xaa-Xaa-Tyr-Xaa-Gln-Xaa-Val-Xaa-Asn-Xaa (SEQ ID NO: 10)
Asn-Gly-Xaa-Xaa-Tyr-Xaa-Gln-Xaa-Val-Xaa-Asn-Xaa (SEQ ID NO: 11)

To the amino group at the N-terminus of the above peptide, a polar substance or a nonpolar substance may be bound to alter the polarity of the molecule; and polyethylene glycol, or glycosaminoglycan (hyaluronic acid and the like) polymer may be bound to confer enzymatic resistance to the peptide. Alternatively, the amino acid may be bound to a liposome substrate for encapsulation into the liposome, or may be immobilized on the surface of a lipid membrane.

Further, the C-terminus of the above peptide may be either -OH or -NH₂.

Specific examples of the peptide include PACAP38 (peptide 1: SEQ ID NO: 12), PACAP27 (peptide 2: SEQ ID NO: 13), VIP (peptide 3: SEQ ID NO: 14), derivatives of PACAP that are Arg-PACAP38 (peptide 4; SEQ ID NO: 15), PACAP-like-peptide (peptide 5; SEQ ID NO: 16), Arg-PACAP27 (peptide 6; SEQ ID NO: 17), [A⁴, V⁵]-PACAP27 (peptide 7; SEQ ID NO: 18) and [A⁴, V⁵, N⁹]-PACAP27 (peptide 8; SEQ ID NO: 19), derivatives of VIP that are [G⁴, I⁵]-VIP (peptide 9; SEQ ID NO: 20) and [G⁴, I⁵, S⁹]-VIP (peptide 10; SEQ ID NO: 21), highly basic derivatives of VIP that are VIP highly basic derivative A (peptide 11; SEQ ID NO: 22), VIP highly basic derivative B (peptide 12; SEQ ID NO: 23), VIP highly basic derivative C (peptide 13; SEQ ID NO: 24) and VIP highly basic derivative D (peptide 14; SEQ ID NO: 25), highly basic derivatives of PACAP27 that are PACAP27 highly basic derivative A (peptide 15; SEQ ID NO: 26), PACAP27 highly basic derivative B (peptide 16; SEQ ID NO: 27) PACAP27 highly basic derivative C (peptide 17; SEQ ID NO: 28), VIP/PACAP highly basic chimera peptide (peptide 18; SEQ ID NO: 29), PACAP shortform (peptide 19 and 26 to 38; SEQ ID NO: 30 and 37 to 49), VIP short form (peptide 20 and 22 to 25; SEQ ID NO: 31 and 33 to 36), PACAP derivative PACAP-like-peptide (stingray-PACAP) (peptide 21; SEQ ID NO: 32) and acetylated PACAP27 basic derivative C (peptide 39; acetylated derivative of SEQ ID NO: 17).

The neuropeptide of the present invention, which has a neuroprotective action and can be used for treating and/or preventing conformational disease, can be synthesized according to any known standard method of peptide synthesis. For example, according to the description in "The Peptides" (Schreder and Luhke, vol. 1, Academic Press, New York, U.S.A., 1966), or in "Peptide Synthesis (Peptide no go-sei)" (Izumiya, et al., MARUZEN, 1975), the neuropeptide can be synthesized by various methods. Specific examples of the method include the azide method, acid chloride method, acid anhydride method, mixed acid anhydride method, DCC method, active ester method (P-nitrophenylester method, N-hydroxysuccinimide ester method, cyanomethyl ester method and the like), any method using Woodward's Reagent K, carboimidazole method, oxidation-reduction method, DCC-additive (HONB, HOBt, HONSu) method and the like. These methods can be applied to both solid phase and liquid phase synthesis.

Peptide synthesis in the present invention is performed by any general polypeptide synthesis method as described above, for example by a so-called stepwise method which involves sequential binding by condensation of an amino acid one by one to the terminal amino acid, or any method which involves the coupling of several divided fragments.

For example, solid phase synthesis by the stepwise method can be specifically performed according to the method of Merrifield. R. B. [Solid phase peptide synthesis, J. Amer. Chem. Soc., 85, 2149-2159 (1963)], as described below. First, a C-terminal amino acid (with a protected amino group) is bound via its carboxyl group to an insoluble resin, and then the protecting group of the amino group of the C-terminal amino acid is removed. Next, to the thus obtained free reactive amino group, according to the amino acid sequence of a target peptide, the reactive carboxyl group of an amino acid with a protected amino group is coupled in order by condensation reaction. After the entire sequence is synthesized stepwise as described above, the peptide is cleaved from the insoluble resin.

Any insoluble resin may be used in the above solid phase synthesis, as long as it has affinity for a reactive carboxyl group. Examples of such an insoluble resin include: chloromethyl resin, oxymethyl resin, 4-oxymethyl phenylacetamide methyl resin (PAM resin), benzhydrylamine resin (BHA resin), aminomethyl resin, methyl benzhydryl resin (MBHA resin), 4-aminomethyl phenoxymethyl resin and 4-hydroxymethyl phenoxymethyl resin.

Further, when 9-fluorenylmethyloxycarbonyl group (Fmoc) is used as a protecting group for an α-amino group, 4-hydroxymethyl phenoxymethyl resin or the like, which can be eliminated from a resin by means of trifluoroacetic acid (TFA), is preferred. When t-butoxycarbonyl group (Boc) is used as the protecting group 4-oxymethyl phenyl acetamido methyl resin (PAM resin) or the like, which can be eliminated from a resin by means of hydrogen fluoride or the like, is preferred.

Peptide concentration per 1 g of resin is preferably 0.5 mmole or less.

In the above method, the binding and elimination of a protecting group to/from an amino group that is involved in peptide bonding of amino acids, and the activation of a carboxyl group that is involved in the peptide bonding of amino acids are required.

Examples of protecting groups for amino groups include benzyloxycarbonyl (Z), t-butoxycarbonyl (Boc), t-aminooxy carbonyl (Aoc), isobonyl oxycarbonyl, p-methoxybenzyloxycarbonyl, 2-chlor-benzyloxycarbonyl, adamantyloxycarbonyl, trifluoroacetyl, phthaloyl, formyl, o-nitrophenylsulphenyl and diphenylphosphino thioyl.

Furthermore, among amino acids, the functional groups on the side chains of, for example His, Tyr, Thr, Lys, Asp, Arg and Ser are preferably protected a priori. Functional groups are protected by binding the protecting groups, as described below, to the above amino acids according to any generally employed method. After reaction, the protecting group is eliminated.

Examples of a protecting group for the imino group of His include benzyloxymethyl (Bom), p-toluenesulphonyl (Tos), benzyl (Bzl), benzyloxycarbonyl (Z) and trityl groups.

The hydroxyl group of Ser and Thr can be protected, for example by means of esterification or etherification, but this protection is not essential. Examples of a group appropriate for esterification include a lower alkanoyl group such as acetyl, an aroyl group such as benzoyl, and any group induced from carbonic acid such as the benzoyloxy carbonyl and ethyloxy carbonyl groups. Further, examples of a group appropriate for etherification include the benzyl (Bzl), tetrahydropyranyl and tert-butyl groups.

Examples of a protecting group for the hydroxyl group of Tyr include the benzyl (Bzl), bromo-benzyloxycarbonyl (Br-Z), dichlorobenzyl (Cl₂-Bzl), benzyloxycarbonyl (Z), acetyl and p-toluenesulphonyl (Tos) groups.

Examples of a protecting group for the amino group of Lys include the benzyloxycarbonyl (Z), chlorobenzyloxy carbonyl (Cl-Z), dichlorobenzyl (Cl₂-Bzl), t-butoxycarbonyl group (Boc) and p-toluenesulphonyl (Tos) groups.

Examples of a protecting group for the guanidino group of Arg include the p-toluenesulphonyl (Tos), nitro, benzyloxycarbonyl (Z) and t-amyloxycarbonyl (Aoc) groups.

The carboxyl group of Asp is protected by, for example esterification by benzyl alcohol, methanol, ethanol, tert-butanol, cyclohexyl (cHex) or the like.

Examples of protecting groups for other amino acids, specifically, examples of that for the indolyl group of Trp include formyl, carbobenzoxyl, 4-methoxy-2,3,6-trimethylbenzenesulfonyl and 2,2,2-trichloroethyloxycarbonyl. However, this protection is not essential.

To protect the thiomethyl group of Met, there is a method, which initially converts the thiomethyl group into methylsulfoxide and then consequently reduces the intermediate products. However, this protection is not essential.

At the same time, carboxyl groups can be activated by any known standard method, and the reagent and the like to be used herein can also be appropriately selected from known reagents and the like. For example, a carboxyl group can be activated by allowing the carboxyl group to react with various reagents to form the corresponding acid chloride, acid anhydride or mixed acid anhydride, azide, active ester (ester with pentachlorophenol, p-nitrophenol, N-hydroxy succinimide, N-hydroxybenztriazole, N-hydroxy-5-norbornen-2,3-dicarboximide or the like) or the like.

Examples of a solvent to be used for the above condensation reaction (peptide bond formation reaction) between a reactive amino group and a reactive carboxyl group in the solid phase may be any solvent that can be used to form peptide bonds. For example, anhydrous or hydrous dimethylformamide (DMF), dimethylsulfoxide (DMSO), pyridine, chloroform, dioxane, dichloromethane (DCM), tetrahydrofuran (THF), ethyl acetate, N-methylpyrrolidone and hexamethylphosphoric triamide (HMPA) can be used alone or as a mixed solvent containing two or more types of these solvents.

Further, the above condensation reaction can be performed in the presence of a condensation agent, for example a carbodiimide reagent such as dicyclohexylcarboxy imide (DCC) or carobodiimidazole, tetraethylpyrophosphate or benzotriazol-N-hydroxy tris dimethylamino phosphonium hexafluorophosphate (Bop reagent).

The thus obtained peptide can be desalted and purified according to any standard method. Examples of such a method include ion exchange chromatography such as DEAE-cellulose, partition chromatography such as Sephadex LH-20 and Sephadex G-25, normal phase chromatography such as silica gel, and reversed-phase chromatography such as ODS-silica gel and high performance liquid chromatography.

The purified peptide obtained as described above can be prepared by using various acids into a pharmacologically acceptable salt if desired, such as acetate, hydrochloride and phosphate.

An agent containing, as active ingredients, the peptide prepared as described above and a pharmacologically acceptable salt thereof has a neuroprotective action against a specific protein that can induce conformational disease. Here, the specific protein that can cause the onset of conformational disease refers to proteins that form insoluble accumulations when an abnormality occurs in the folding of the protein due to an abnormality of the gene, and forms a β-sheet structure differing from the conformation that the original protein can take. Further, a conformational disease refers to a disease, wherein when cell death occurs among neurons because of the accumulation of the above specific protein, symptoms characterized by progressive dementia, myoclonus, ataxia, dysarthria or the like are induced. Examples of specific proteins and conformational diseases caused by the same include: Creutzfeldt-Jakob disease, mad cow disease (cattle spongy encephalopathy) and Gerstmann-Straussler syndrome by prion protein; bulbar spinal muscular atrophy, Huntington's disease, spinocerebellar ataxia and Dentatorubral Pallidoluysian atrophy by polyglutamine; Parkinson disease, Lewy Body Dementia and polyphyletic atrophy by α-Synuclein; Alzheimer disease and Down's syndrome by β-amyloid; familial amyotrophic lateral sclerosis by SOD1; FTDP-17, progressive supranuclear palsy, cortical-basal ganglia degeneration and Pick's disease by Tau; familial British dementia by Abri; and familial dementia by neuroserpin. Further, neuroprotective action refers to an action that can inhibit cell death among neurons, due to specific proteins that can cause the onset of the above conformational diseases, so as to maintain the role of the neurons. It is said that cell death due to apoptosis is caused by the process of the development of the conformational disease. Actually, there is a report about such an action on the caspase system that is deeply involved in apoptosis [Journal of neuroscience research (2001) 65, 45-53]. For example, this action is examined by adding a toxic protein such as prion to test subject cells to stimulate the cells in the presence of a test substance having a neuroprotective action against the toxic protein, detecting the activity of caspase, which is a marker substance for cell death, and then comparing the caspase activity with that of controls wherein the test substance is absent. When the activity in the subject cells is lower than that of the control, the test substance can be determined as having a neuroprotective action against the specific protein. In addition, similar assessment can also be made by comparing viable cell counts instead of detecting caspase activity in the above test.

The peptide of the present invention and derivatives thereof have a neuroprotective action, and each derivative is observed to have stronger activity and a clinically desirable supportive effect, compared with PACAP and VIP. For example, a basic amino acid substitution derivative group including Arg-PACAP38 and Arg-PACAP27 has extremely strong resistance against peptidase in vivo, and this is specified in Peptide Chemistry (1996) 249-252. Generally, peptide and protein are immediately metabolized in vivo, and an important substance that takes a part in this metabolic process is peptidase. Hence, resistance against the peptidase leads to a prolonged action time of the basic amino acid substitution derivative group in vivo, so that it is very effective against conformational diseases, which is a chronic disease. This result brings a reduction in dosage frequency accompanied by a decrease in drug price, and can be considered as having significant effects even when medically and economically considered. Furthermore, a derivative group whose N-terminus has been altered do not show PACAP's transient action of contracting the smooth muscle of the respiratory system. Thus, a wider range of possible subject patients can benefit from its clinical application. The above derivative having supportive effects shows a neuroprotective action superior to that of PACAP and VIP. In particular, among PACAP derivatives, Arg-PACAP38 (peptide 4; SEQ ID NO: 16), Arg-PACAP27 (peptide 6; SEQ ID NO: 18), PACAP27 highly basic derivative A (peptide 15: SEQ ID NO: 26), PACAP27 highly basic derivative B (peptide 16: SEQ ID NO: 27), and PACAP27 highly basic derivative C (peptide 17: SEQ ID NO: 28), and among VIP derivatives, [G⁴, I⁵, S⁹]-VIP (peptide 10; SEQ ID NO: 22) show significant action.

The agent of the present invention containing, as active ingredients, the peptide and a pharmacologically acceptable salt thereof is effective for treating and preventing conformational diseases such as Down's syndrome, Parkinson disease, lewy bodies dementia, polyphyletic atrophy, Creutzfeldt-Jakob disease, mad cow disease (cattle spongy encephalopathy), Alzheimer disease, Gerstmann-Straussler syndrome, bulbar spinal muscular atrophy, Huntington's disease, spinocerebellar ataxia, Dentatorubral Pallidoluysian atrophy, familial amyotrophic lateral sclerosis, FTDP-17, progressive supranuclear palsy, cortical-basal ganglia degeneration, Pick's disease, familial British dementia, familial dementia and the like.

When the peptide and the salt thereof of the present invention are used for treating a patient with the above disease, they can be administered intact.

Furthermore, the peptide and the salt thereof of the present invention are mixed with various pharmacologically acceptable carriers and other components by any known method, and then the mixture can be used as a liquid or solid pharmaceutical composition for use in parenteral, oral, aerosol, transpulmonary, transcutaneous, transnasal or eye drop forms to act on the central nervous system. For example, the composition can be safely administered orally or parenterally (for example, local, rectal and intravenous administration) in the form of a tablet (including a sugar-coated tablet and film coated tablet), powder, granule, capsule (including a soft capsule), liquid drug, injection, suppository, sustained-release agent or the like. In addition, the pharmaceutical composition of the present invention can directly target the brain by means of an intraventricular pump.

Examples of a pharmacologically acceptable carrier to be used in the pharmaceutical composition of the present invention include various organic or inorganic carrier substances that are commonly used as materials for pharmaceutical preparations. For example, excipients, lubricants, binders and disintegrators are used in solid pharmaceutical preparations; solvents, solubilizers, suspending agents, isotonizing agents, buffers and soothing agents are used in liquid pharmaceutical preparations. In addition, additives such as antiseptic, anti-oxidants, colorants, sweetening agents, adsorbates, and moistening agents can be used if necessary. As an excipient, for example, lactose, saccharose, D-mannitol, starch, cornstarch, crystalline cellulose or light anhydrous silicic acid is used. As a lubricant, for example, magnesium stearate, calcium stearate, talc or colloidal silica is used. Examples of a binder include crystalline cellulose, saccharose, D-mannitol, dextrin, hydroxypropylcellulose, hydroxypropylmethylcellulose, polyvinylpyrrolidone, starch, sucrose, gelatine, methylcellulose and sodium carboxymethylcellulose. Examples of a disintegrating agent include starch, carboxymetylcellulose, carboxymetylcellulose calcium, crosscarmellose sodium, sodium carboxymethyl starch and L-hydroxypropylcellulose. Examples of a solvent include water for injection, alcohol, propylene glycol, macrogol, sesame oil and corn oil. Examples of a solubilizer include polyethylene glycol, propylene glycol, D-mannitol, benzyl benzoate, ethanol, tris aminomethane, cholesterol, triethanolamine, sodium carbonate and sodium citrate. As a suspending agent, for example, a surfactant such as stearyl triethanolamine, sodium lauryl sulfate, lauryl aminopropionic acid, lecithin, benzalkonium chloride, benzethonium chloride and glyceryl monostearate; or a hydrophilic macromolecule such as polyvinyl alcohol, polyvinylpyrrolidone, sodium carboxymethylcellulose, methylcellulose, hydroxymethylcellulose and hydroxyethylcellulose, hydroxypropylcellulose is used. Examples of an isotonizing agent include glucose, D-sorbitol, sodium chloride, glycerine and D-mannitol. Examples of a buffer include phosphate, acetate, carbonate, and citrate. An example of a soothing agent is benzyl alcohol or the like. Examples of an antiseptic include p-oxybenzoic acid esters, chlorobutanol, benzyl alcohol, phenethyl alcohol, dehydroacetic acid and sorbic acid. Examples of an anti-oxidant include sulfite and ascorbic acid.

The dosage of the peptide or the salt thereof of the present invention is appropriately determined depending on the purpose of use (test), age of subject, body weight, administration method and the like. For example, when intravenous administration is performed for mammals including humans, preferably 0.1 to 100 µg of peptide is used as dosage per administration.

### EXAMPLE

The present invention will be explained more specifically by examples to be described below, but the present invention is not limited to these examples.

### [Example 1] (Peptide synthesis)

PACAP38, PACAP27, VIP (peptides 1 to 3) and peptide derivatives (peptides 4 to 39) induced from these peptides having the following amino acid sequences were respectively synthesized according to general peptide solid phase synthesis methods. Unless specified, the N-terminus and C-terminus of each peptide are -H and -NH₂, respectively.
(1) Peptide 1: PACAP38 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala-Val-Leu-Gly-Lys-Arg-Tyr-Lys-Gln-Arg-Val-Lys-Asn-L
ys (SEQ ID NO: 12)
(2) Peptide 2: PACAP27 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala-Val-Leu (SEQ ID NO: 13)
(3) Peptide 3: VIP 
His-Ser-Asp-Ala-Val-Phe-Thr-Asp-Asn-Tyr-Thr-Arg-Leu-Arg-Lys-Gln-Met-Ala-V
al-Lys-Lys-Tyr-Leu-Asn-Ser-Ile-Leu-Asn (SEQ ID NO: 14)
(4) Peptide 4: PACAP derivative Arg-PACAP38 
His-Ser-Asp-Gly-Ile-Phe-Thr-Asp-Ser-Tyr-Ser-Arg-Tyr-Arg-Arg-Gln-Leu-Ala-Val
-Arg-Arg-Tyr-Leu-Ala-Ala-Val-Leu-Gly-Arg-Arg-Tyr-Arg-Gln-Arg-Val-Arg-Asn-
Arg (SEQ ID NO: 15)
(5) Peptide 5: PACAP derivative PACAP-like-peptide (stargazer-PACAP) 
His-Ser-Asp-Gly-Ile-Phe-Thr-Asp-Ser-Tyr-Ser-Arg-Tyr-Arg-Lys-Gln-Met-Ala-Val
-Gln-Lys-Tyr-Leu-Ala-Ala-Val-Leu-Gly-Arg-Arg-Tyr-Arg-Gln-Arg-Val-Arg-Asn-
Lys (SEQ ID NO: 16)
(6) Peptide 6: PACAP derivative Arg-PACAP27 
His-Ser-Asp-Gly-Ile-Phe-Thr-Asp-Ser-Tyr-Ser-Arg-Tyr-Arg-Arg-Gln-Leu-Ala-Val
-Arg-Arg-Tyr-Leu-Ala-Ala-Val-Leu (SEQ ID NO: 17)
(7) Peptide 7: PACAP derivative [A⁴, V⁵]-PACAP27 
His-Ser-Asp-Ala-Val-Phe-Thr-Asp-Ser-Tyr-Ser-Arg-Tyr-Arg-Lys-Gln-Met-Ala-Va
l-Lys-Lys-Tyr-Leu-Ala-Ala-Val-Leu (SEQ ID NO: 18)
(8) Peptide 8: PACAP derivative [A⁴,V⁵, N⁹]-PACAP27 
His-Ser-Asp-Ala-Val-Phe-Thr-Asp-Asn-Tyr-Ser-Arg-Tyr-Arg-Lys-Gln-Met-Ala-V
al-Lys-Lys-Tyr-Leu-Ala-Ala-Val-Leu (SEQ ID NO: 19)
(9) Peptide 9: VIP derivative [G⁴, I⁵]-VIP 
His-Ser-Asp-Gly-Ile-Phe-Thr-Asp-Asn-Tyr-Thr-Arg-Leu-Arg-Lys-Gln-Met-Ala-V
al-Lys-Lys-Tyr-Leu-Asn-Ser-Ile-Leu-Asn (SEQ ID NO: 20)
(10) Peptide 10: VIP derivative [G⁴, I⁵, S⁹]-VIP 
His-Ser-Asp-Gly-Ile-Phe-Thr-Asp-Ser-Tyr-Thr-Arg-Leu-Arg-Lys-Gln-Met-Ala-Va
l-Lys-Lys-Tyr-Leu-Asn-Ser-Ile-Leu-Asn (SEQ ID NO: 21)
(11) Peptide 11: VIP highly basic derivative A 
His-Ser-Asp-Ala-Val-Phe-Thr-Asp-Asn-Tyr-Thr-Arg-Leu-Arg-Arg-Gln-Leu-Ala-V
al-Arg-Arg-Tyr-Leu-Asn-Ser-Ile-Leu-Asn-Gly-Lys (SEQ ID NO: 22)
(12) Peptide 12: VIP highly basic derivative B 
His-Ser-Asp-Ala-Val-Phe-Thr-Asp-Asn-Tyr-Thr-Arg-Leu-Arg-Arg-Gln-Leu-Ala-V
al-Arg-Arg-Tyr-Leu-Asn-Ser-Ile-Leu-Asn-Gly-Lys-Arg (SEQ ID NO: 23)
(13) Peptide 13: VIP highly basic derivative C 
His-Ser-Asp-Ala-Val-Phe-Thr-Asp-Asn-Tyr-Thr-Arg-Leu-Arg-Arg-Gln-Leu-Ala-V
al-Arg-Arg-Tyr-Leu-Asn-Ser-Ile-Leu-Asn-Gly-Arg-Arg (SEQ ID NO: 24)
(14) Peptide 14: VIP highly basic derivative D 
His-Ser-Asp-Ala-Val-Phe-Thr-Asp-Asn-Tyr-Thr-Arg-Leu-Arg-Arg-Gln-nLeu-Ala-
Val-Arg-Arg-Tyr-Leu-Asn-Ser-Ile-Leu-Asn-Gly-Arg-Arg (SEQ ID NO: 25)
(15) Peptide 15: PACAP27 highly basic derivative A 
His-Ser-Asp-Gly-Ile-Phe-Thr-Asp-Ser-Tyr-Ser-Arg-Tyr-Arg-Arg-Gln-Leu-Ala-Val
-Arg-Arg-Tyr-Leu-Ala-Ala-Val-Leu-Gly-Lys (SEQ ID NO: 26)
(16) Peptide 16: PACAP27 highly basic derivative B 
His-Ser-Asp-Gly-Ile-Phe-Thr-Asp-Ser-Tyr-Ser-Arg-Tyr-Arg-Arg-Gln-Leu-Ala-Val
-Arg-Arg-Tyr-Leu-Ala-Ala-Val-Leu-Gly-Lys-Arg (SEQ ID NO: 27)
(17) Peptide 17: PACAP27 highly basic derivative C 
His-Ser-Asp-Gly-Ile-Phe-Thr-Asp-Ser-Tyr-Ser-Arg-Tyr-Arg-Arg-Gln-Leu-Ala-Val
-Arg-Arg-Tyr-Leu-Ala-Ala-Val-Leu-Gly-Arg-Arg (SEQ ID NO: 28)
(18) Peptide 18: VIP/PACAP highly basic chimera peptide 
His-Ser-Asp-Ala-Val-Phe-Thr-Asp-Asn-Tyr-Thr-Arg-Leu-Arg-Arg-Gln-nLeu-Ala-
Val-Arg-Arg-Tyr-Leu-Asn-Ser-Ile-Leu-Asn-Gly-Arg-Arg-Tyr-Arg-Gln-Arg-Val-Ar
g-Asn-Arg (SEQ ID NO: 29)
(19) Peptide 19: PACAP short form 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu (SEQ ID NO: 30)
(20) Peptide 20: VIP short form 
His-Ser-Asp-Ala-Val-Phe-Thr-Asp-Asn-Tyr-Thr-Arg-Leu-Arg-Lys-Gln-Met-Ala-V
al-Lys-Lys-Tyr-Leu (SEQ ID NO: 31)
(21) Peptide 21: PACAP derivative PACAP-like-peptide (stingray-PACAP) 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala-Val-Leu-Gly-Lys-Arg-Tyr-Lys-Pro-Lys-Arg-Arg-Asn-S
er-Gly-Arg-Arg-Val-Phe-Tyr (SEQ ID NO: 32)
(22) Peptide 22: VIP short form (VIP24) 
His-Ser-Asp-Ala-Val-Phe-Thr-Asp-Asn-Tyr-Thr-Arg-Leu-Arg-Lys-Gln-Met-Ala-V
al-Lys-Lys-Tyr-Leu-Asn (SEQ ID NO: 33)
(23) Peptide 23: VIP short form (VIP25) 
His-Ser-Asp-Ala-Val-Phe-Thr-Asp-Asn-Tyr-Thr-Arg-Leu-Arg-Lys-Gln-Met-Ala-V
al-Lys-Lys-Tyr-Leu-Asn-Ser (SEQ ID NO: 34)
(24) Peptide 24: VIP short form (VIP26) 
His-Ser-Asp-Ala-Val-Phe-Thr-Asp-Asn-Tyr-Thr-Arg-Leu-Arg-Lys-Gln-Met-Ala-V
al-Lys-Lys-Tyr-Leu-Asn-Ser-Ile (SEQ ID NO: 35)
(25) Peptide 25: VIP short form (VIP27) 
His-Ser-Asp-Ala-Val-Phe-Thr-Asp-Asn-Tyr-Thr-Arg-Leu-Arg-Lys-Gln-Met-Ala-V
al-Lys-Lys-Tyr-Leu-Asn-Ser-Ile-Leu (SEQ ID NO: 36)
(26) Peptide 26: PACAP short form (PACAP24) 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala (SEQ ID NO: 37)
(27) Peptide 27: PACAP short form (PACAP25) 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala (SEQ ID NO: 38)
(28) Peptide 28: PACAP short form (PACAP26) 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala-Val (SEQ ID NO: 39)
(29) Peptide 29: PACAP long form (PACAP28) 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala-Val-Leu-Gly (SEQ ID NO: 40)
(30) Peptide 30: PACAP long form (PACAP29) 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala-Val-Leu-Gly-Lys (SEQ ID NO: 41)
(31) Peptide 31: PACAP long form (PACAP30) His-Ser-Asp-Gly-Ile-Phe-Thr-Asp-Ser-Tyr-Ser-Arg-Tyr-Arg-Lys-Gln-Met-Ala-Val -Lys-Lys-Tyr-Leu-Ala-Ala-Val-Leu-Gly-Lys-Arg (SEQ ID NO: 42)
(32) Peptide 32: PACAP long form (PACAP31) 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala-Val-Leu-Gly-Lys-Arg-Tyr (SEQ ID NO: 43)
(33) Peptide 33: PACAP long form (PACAP32) 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala-Val-Leu-Gly-Lys-Arg-Tyr-Lys (SEQ ID NO: 44)
(34) Peptide 34: PACAP long form (PACAP33) 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala-Val-Leu-Gly-Lys-Arg-Tyr-Lys-Gln (SEQ ID NO: 45)
(35) Peptide 35: PACAP long form (PACAP34) 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala-Val-Leu-Gly-Lys-Arg-Tyr-Lys-Gln-Arg (SEQ ID NO:
46)
(36) Peptide 36: PACAP long form (PACAP35) 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala-Val-Leu-Gly-Lys-Arg-Tyr-Lys-Gln-Arg-Val (SEQ ID
NO: 47)
(37) Peptide 37: PACAP long form (PACAP36) 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala-Val-Leu-Gly-Lys-Arg-Tyr-Lys-Gln-Arg-Val-Lys (SEQ
ID NO: 48)
(38) Peptide 38: PACAP long form (PACAP37) 
His-Ser-Asp-Gly-Ile-Phe-Thr-Asp-Ser-Tyr-Ser-Arg-Tyr-Arg-Lys-Gln-Met-Ala-Val
-Lys-Lys-Tyr-Leu-Ala-Ala-Val-Leu-Gly-Lys-Arg-Tyr-Lys-Gln-Arg-Val-Lys-Asn
(SEQ ID NO: 49)
(39) Peptide 39: Acetyl-PACAP27 highly basic derivative C
   Acetyl-His-Ser-Asp-Gly-Ile-Phe-Thr-Asp-Ser-Tyr-Ser-Arg-Tyr-Arg-Arg-Gln-Leu-Ala-Val-Arg-Arg-Tyr-Leu-Ala-Ala-Val-Leu-Gly-Arg-Arg (acetylated derivative of peptide 17)

### [Example 2] Examination of cell death by abnormal prion (106-126)

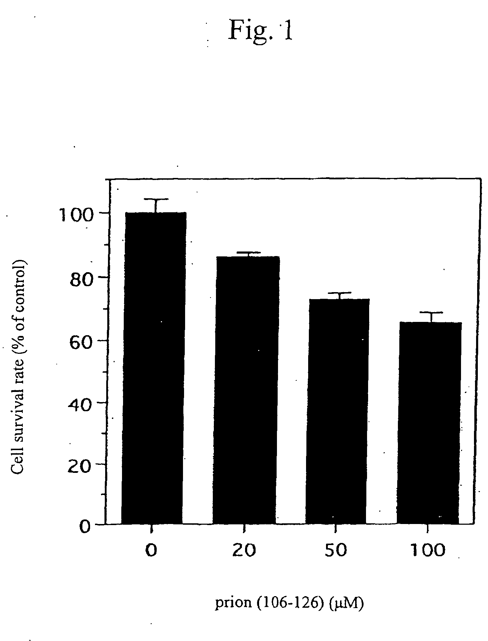

It has been already confirmed that normal prion is converted to an abnormal form by conformational conversion (J. Neurochem, (2000) vol.75, 2536-2545). Accordingly, cell death by the prion protein (106-126) with abnormal folding was examined using PC 12 cells, which are cells derived from rat adrenal pheochromocytoma and used as a model cell line in brain/nervous system research. In a 5% horse serum and 5% newborn calf serum-containing Dulbecco's modified minimum essential medium (DMEM), PC 12 cells were cultured at a constant temperature at 37°C under an environment of 5% CO₂ and 95% air. After PC 12 cells were removed from the culture flask using trypsin, the above cells were cultured on a 96-well culture plate (10⁴ cells/well). Prion (106-126) (American Peptide Company) was added to the cultured PC12 cells, so that the effect was confirmed. Prion (106-126) had been pre-treated at a concentration of approximately 5 mg/mL for approximately 72 hours, and then converted to aggregates (fibrils changed to have β-sheet structure). Cell death was monitored by detecting the amount of lactate dehydrogenase released from the cells to the medium. Figure 1 shows the result. It was clearly shown that cell death occurs depending on the concentration of an abnormal prion (106 -126).

### [Example 3] Neuroprotective action of PACAP and VIP against abnormal prion (106-126)

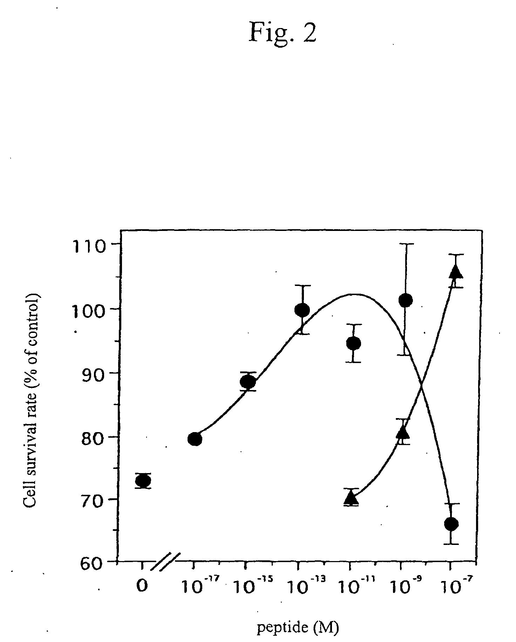

50 µM abnormal prion (106-126) and neuropeptides (PACAP and VIP) at various concentrations were added to PC12 cells that had been cultured similarly to Example 2. Cell count was calculated by the WST-8 method, so that the degree of neuroprotective action against abnormal prion (106-126) was expressed numerically. Figure 2 shows the results. It was confirmed that even at an extremely low concentrations, PACAP showed a certain degree of neuroprotective action. In contrast, VIP showed neuroprotective action at concentrations higher than that of PACAP. In addition, this neuroprotective effect was confirmed in 10⁻⁹ M of each of all neuropeptides I to 21.

### [Example 4] Examination of cell death by β-amyloid

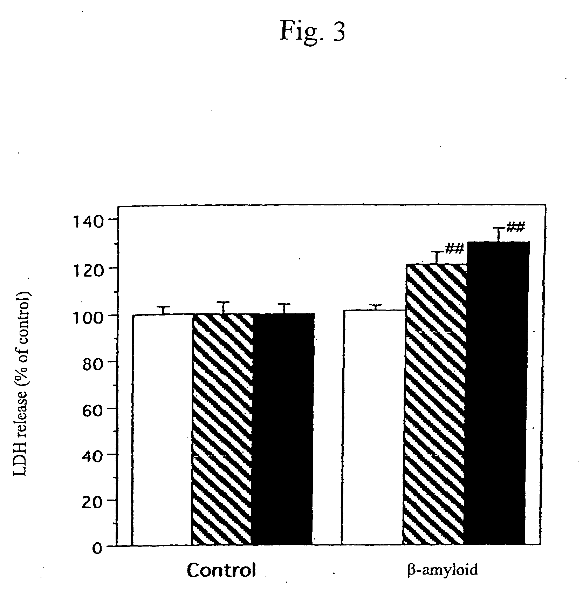

β-amyloid (β-amyloid 1-42, American Peptide Company) (10⁻³ M) was added to PC12 cells that had been cultured similarly to Example 2. After 48 hours of culturing, the resulting cell death was examined. β-amyloid had been pre-treated at a concentration of approximately 5 mg/mL for approximately 24 hours, so as to be converted to aggregates (fibrils). Cell death was monitored by detecting lactate dehydrogenase that is normally present on cell membranes and is released outside when the cell membranes are disrupted accompanying cell death. Cell death induced by β-amyloid was examined for each of three types of culture media, namely 5% horse serum and 5% newborn calf serum-containing DMEM, 0.25% horse serum and 0.25% newborn calf serum-containing DMEM, and 0.2 µM insulin-containing serum-free DMEM. Figure 3 shows the results. In 0.2 µM insulin-containing serum-free and 0.25% horse serum and 0.25% newborn calf serum-containing DMEM, compared with a control group (no β-amyloid-added group), significant cell death by β-amyloid was confirmed. On the other hand, no cell death by β-amyloid was confirmed for PC12 culture cells in the 5% horse serum and 5% newborn calf serum-containing DMEM, even after 48 hours. These results suggested the possibility that serum components are involved in some kind of neuroprotection. In addition, based on the above results, it was determined to use 0.2 µM insulin-containing serum-free DMEM in the examples that follow.

### [Example 5] Neuroprotective action of neuropeptide PACAP27 against β-amyloid

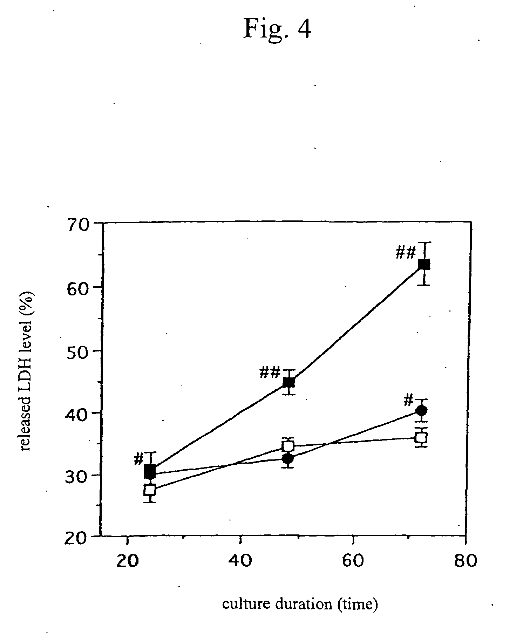

β-amyloid was added to PC12 cells that had been cultured similarly to Example 4. At this time, neuropeptide PACAP27 was allowed to co-exist, so that the neuroprotective action against cell death by β-amyloid was confirmed. Cell death was monitored by detecting the amount of lactate dehydrogenase released from the cells into the media. Figure 4 shows the results. It could be confirmed that the cells died over time in the presence of β-amyloid. Further, not many cells died after 24 hours of culturing, but died after the passage of a certain period of time, suggesting that cell death by β-amyloid may be due to apoptosis induction. In contrast, cell death was very strongly suppressed in the cell group added with neuropeptide PACAP27, such that the value of cell death was almost the same value as that of the control. These results revealed that PACAPs show extremely strong neuroprotective action against β-amyloid.

### [Example 6] Neuroprotective action of various neuropeptides against amyloid

An experiment was performed similarly to Example 5, and then the neuroprotective action against cell death by β-amyloid of the following PACAP-related neuropeptides (peptides 1, 2, 4, 5, 7, 8, 15, 16, 17, 19, 21, 26, 27, 28, 29, 30, 31, 32, 33, 34, 35, 36, 37, 38 and 39) and VIP-related neuropeptides (peptides 3, 9, 10, 11, 12, 13, 14, 18, 20, 22, 23, 24 and 25) was confirmed. Specifically, amyloid (50 µM) and each of the various PACAP-related neuropeptides and VIP-related neuropeptides were added to the cultured PC12 cells. The number of iving cells was calculated by the WST-8 method, so that comparison and examination was performed for the neuroprotective action of these various peptides against amyloid. If the neuropeptides had neuroprotective action, a clear difference in viable cell count can be observed between cases in which neuropeptides were added and cases in which neuropeptides were not added. In PC12 cells added with each of the various neuropeptides, a case wherein a higher cell survival rate (P<0.05) was observed compared with groups in which neuropeptides had not been added, was determined as having neuroprotective action. The results are shown in Table 1 and Table 2.

**Table 1**

| PACAP-related neuropeptide (10⁻⁹ M) | | | |
|---|---|---|---|
| Peptide added | Neuroprotective action | Peptide added | Neuroprotective action |
| No peptide added | - | Peptide 27 | + |
| Peptide 1 | + | Peptide 28 | + |
| Peptide 2 | + | Peptide 29 | + |
| Peptide 4 | + | Peptide 30 | + |
| Peptide 5 | + | Peptide 31 | + |
| Peptide 6 | + | Peptide 32 | + |
| Peptide 7 | + | Peptide 33 | + |
| Peptide 8 | + | Peptide 34 | + |
| Peptide 15 | + | Peptide 35 | + |
| Peptide 16 | + | Peptide 36 | + |
| Peptide 17 | + | Peptide 37 | + |
| Peptide 19 | + | Peptide 38 | + |
| Peptide 21 | + | Peptide 39 | + |
| Peptide 26 | + | | |
| (+, *P*<0.05 when compared with the no neuropeptide-added group) | | | |

**Table 2**

| VIP-related neuropeptide (10⁻⁷ M) | | | |
|---|---|---|---|
| Peptide added | Neuroprotective action | Peptide added | Neuroprotective action |
| No peptide added | - | Peptide 14 | + |
| Peptide 3 | + | Peptide 18 | + |
| Peptide 9 | + | Peptide 20 | + |
| Peptide 10 | + | Peptide 22 | + |
| Peptide 11 | + | Peptide 23 | + |
| Peptide 12 | + | Peptide 24 | + |
| Peptide 13 | + | Peptide 25 | + |
| (+, *P*<0.05 when compared with the no neuropeptide-added group) | | | |

### [Example 7] Neuroprotective action of neuropeptides PACAP27, VIP and humanin, and a cell-permeable cAMP derivative

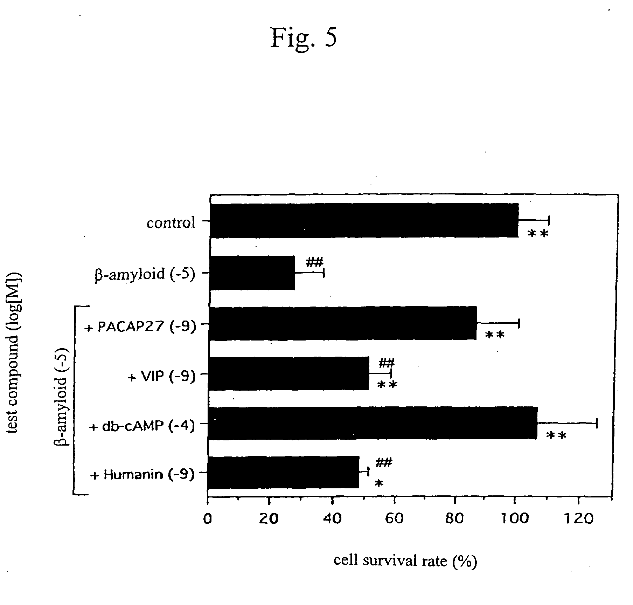

β-amyloid was added to PC12 cells that had been cultured similarly to Example 5. At this time, neuropeptides PACAP27, VIP and humanin, and a cell-permeable cAMP derivative were allowed to respectively co-exist, so that the effect of these neuropeptides against cell death by β-amyloid was confirmed. Cell death was examined by evaluating cell count using the WST-8 method, and then by calculating cell survival rate. Figure 5 shows the results. In the presence of β-amyloid, approximately 70% of the cells died. On the other hand, in the case wherein dibutyryl-cAMP, a cAMP derivative, was allowed to coexist, cell death was very strongly suppressed so that neuroprotective action of camp was confirmed. Further, PACAP27 (10⁻⁹ M) also showed distinctly strong neuroprotective action. PACAPs are known to exhibit the action through a rise in cAMP. These results suggested the possibility that the neuroprotective action of the neuropeptides against β-amyloid may also be exhibited through cAMP. Furthermore, VIP, an analogue of PACAP27, showed neuroprotective action against β-amyloid although the effect was weaker than that of PACAP27. The neuroprotective action of humanin, a recently found human-derived neuroprotective substance (Proc Natl Acad Sci USA (2001) 98 (11) 6336-6341), was also examined. Humanin showed neuroprotective action, although the effect was also weaker than that of PACAP27.

### [Example 7] Relationship between the concentration of neuropeptides and neuroprotective action

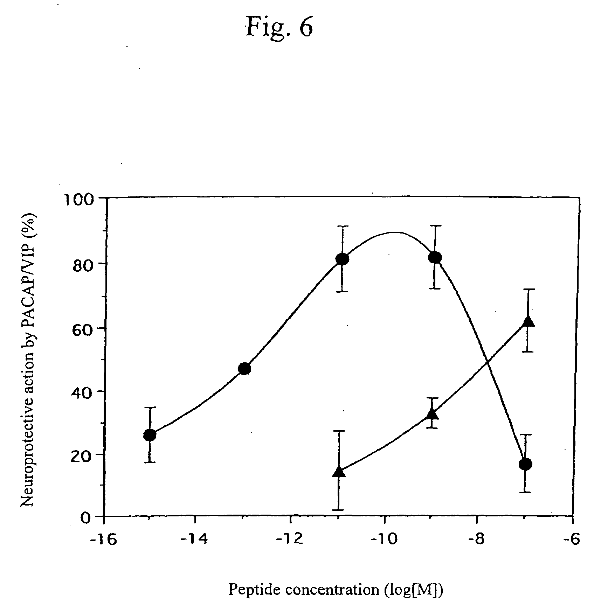

β-amyloid was added to PC 12 cells that had been cultured similarly to Example 5. At this time, neuropeptides at various concentrations were allowed to co-exist, so that the relationship between the neuroprotective action and the concentration was investigated. Cells were calculated by the WST-8 method, and then the extent to which cell death by β-amyloid could be attenuated was expressed numerically. Figure 6 shows the results. Neuropeptide PACAP showed neuroprotective action even at an extremely low concentration, and the effect was strongest at 10⁻⁹ to 10⁻¹³ M. Further, VIP also showed strong neuroprotective action at relatively high concentrations. These results clearly showed that PACAP has neuroprotective action that is at least 1X 10⁴ fold greater than that of VIP.

### [Example 8] Caspase-3 activating action of β-amyloid and the effect of neuropeptide to suppress the action

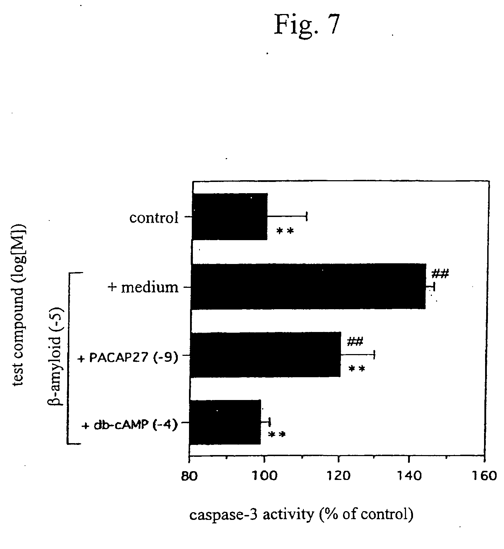

PC12 cells that had been cultured almost similarly to Example 4 were cultured in a 24-well plate (2 x 10⁵ cells/well). 10⁻⁵ M β-amyloid was added to the plate. At this time, cAMP derivative (10⁻⁴ M) and neuropeptide PACAP (10⁻⁹ M) were added respectively, and then the action of activating caspase-3, an apoptosis-related enzyme, was examined. The caspase-3 activating action was examined using Ac-DEVD-AMC, a caspase-3 specific substrate. Figure 7 shows the results. The activity of caspase-3 was improved by the addition of β-amyloid, but the action was significantly attenuated by the addition of the cAMP derivative. Further, by the addition of neuropeptide PACAP, an incomplete, approximately 50% suppressive effect was shown. These results suggested a possibility that the action of the neuropeptides on the caspase system is largely involved in the neuroprotective action against β-amyloid. Taking into consideration that this suppressive action is not complete, other action mechanisms may also be involved.

### Industrial Applicability

The agent and the pharmaceutical composition of the present invention have neuroprotective action, and are effective for treating and/or preventing conformational diseases.

All publications, patents and patent applications cited herein are incorporated herein by reference in their entirety.

## Claims

1. A therapeutic and/or prophylactic agent against conformational diseases, which contains as an active ingredient a neuropeptide having a neuroprotective action or a pharmacologically acceptable salt thereof.

2. The agent according to claim 1, wherein the neuropeptide comprises at least 23 residues from the N terminus of a peptide that is represented by the following formula (I): 
His-Ser-Asp-A-B-Phe-Thr-Asp-C-Tyr-D-Arg-E-Arg-F-Gln-G-Ala-Val-I-J-Tyr-Leu
-K-L-M-Leu-N (I)
 (wherein A represents Ala or Gly; B represents Ile or Val; C represents Asn or Ser; D represents Thr or Ser; E represents Leu or Tyr; F and J respectively represent Lys or Arg; I represents Lys, Arg or Gln; but at least one of F, I and J represents Arg; G represents Met, Leu or nLeu; K represents Asn or Ala; L represents Ser or Ala; M represents Ile or Val; and N represents -NH₂ or Asn-NH₂).

3. The agent according to claim 1, wherein the neuropeptide is represented by the following formula (II): 
His-Ser-Asp-A-B-Phe-Thr-Asp-C-Tyr-D-Arg-E-Arg-F-Gln-G-Ala-Val-I-J-Tyr-Leu
-K-L-M-Leu-P-Gly-Q-R (II)
 (wherein A represents Ala or Gly; B represents Ile or Val; C represents Asn or Ser; D represents Thr or Ser; E represents Leu or Tyr; F and J respectively represent Lys or Arg; I represents Lys, Arg or Gln; but at least one of F, I and J represents Arg; G represents Met, Leu or nLeu; K represents Asn or Ala; L represents Ser or Ala; M represents Ile or Val; P represents Asn or a chemical bond; Q represents Lys, Arg, Lys-Arg, Arg-Arg or a chemical bond; and R represents -OH or -NH₂).

4. The agent according to claim 1, wherein the neuropeptide is represented by the following formula (III): 
His-Ser-Asp-A-B-Phe-Thr-Asp-C-Tyr-D-Arg-E-Arg-F-Gln-G-Ala-Val-I-J-Tyr-Leu
-K-L-M-Leu-N-Gly-O-P-Tyr-Q-Gln-R-Val-S-Asn-T-U (III)
 (wherein A represents Ala or Gly; B represents Ile or Val; C represents Asn or Ser; D represents Thr or Ser; E represents Leu or Tyr; F, J, O, P, Q, R, S and T respectively represent Lys or Arg; I represents Lys, Arg or Gln; but at least one of F, I and J represents Arg; G represents Met, Leu or nLeu; K represents Asn or Ala; L represents Ser or Ala; M represents Ile or Val; N represents a chemical bond or Asn; and U represents -OH or -NH₂).

5. The agent according to any one of claims 1 to 4, wherein the conformational disease is developed by the accumulation of prion protein, polyglutamic acid, α-Synuclein, β-amyloid, SOD1, Tau, Abri or neuroserpin.

6. The agent according to any one of claims 1 to 5, wherein the conformational disease is Creutzfeldt-Jakob disease, mad cow disease (cattle spongy encephalopathy), Gerstmann-Straussler syndrome, bulbar spinal muscular atrophy, Huntington's disease, spinocerebellar ataxia, Dentatorubral Pallidoluysian atrophy, Parkinson disease, Lewy Body Dementia, polyphyletic atrophy, Alzheimer disease, Down's syndrome, familial amyotrophic lateral sclerosis, FTDP-17, progressive supranuclear palsy, cortical-basal ganglia degeneration, Pick's disease, familial British dementia or familial dementia.

7. A pharmaceutical composition, comprising the agent of any one of claims 1 to 6.


SEQUENCE LISTING

<110> ITOHAM FOODS INC.

<120> Pharmaceutical composition for conformation disease

<130> PH-1709-PCT

<150> JP 2001-386699

<151> 2001-12-19

<160> 49

<170> PatentIn Ver. 2.1

<210> 1
<211> 27
<212> PRT
<213> Artificial Sequnece

<220>
<223> Synthetic Peptide

<220>
<221> PEPTIDE
<222> 4
<223> Xaa represents Ala or Gly

<220>
<221> PEPTIDE
<222> 5
<223> Xaa represents Ile or Val

<220>
<221> PEPTIDE
<222> 9
<223> Xaa represents Asn or Ser

<220>
<221> PEPTIDE
<222> 11
<223> Xaa represents Thr or Ser

<220>
<221> PEPTIDE
<222> 13
<223> Xaa represents Leu or Tyr

<220>
<221> PEPTIDE
<222> 15
<223> Xaa represents Lys or Arg

<220>
<221> PEPTIDE
<222> 17
<223> Xaa represents Met or Leu or nLeu

<220>
<221> PEPTIDE
<222> 20
<223> Xaa represents Lys or Arg or Gln

<220>
<221> PEPTIDE
<222> 21
<223> Xaa represents Lys or Arg

<220>
<221> PEPTIDE
<222> 24
<223> Xaa represents Asn or Ala

<220>
<221> PEPTIDE
<222> 25

<223> Xaa represents Ser or Ala

<220>
<221> PEPTIDE
<222> 26
<223> Xaa represents Ile or Val

<400> 1
His Ser Asp Xaa Xaa Phe Thr Asp Xaa Tyr Xaa Arg Xaa Arg Xaa Gln
1               5                   10                  15

Xaa Ala Val Xaa Xaa Tyr Leu Xaa Xaa Xaa Leu
            20                  25


<210> 2
<211> 2
<212> PRT
<213> Artificial Sequnece

<220>
<223> Synthetic Peptide

<400> 2
Gly Lys
1


<210> 3
<211> 2
<212> PRT
<213> Artificial Sequnece

<220>
<223> Synthetic Peptide

<400> 3
Gly Arg

1

<210> 4
<211> 3
<212> PRT
<213> Artificial Sequnece

<220>
<223> Synthetic Peptide

<400> 4
Gly Lys Arg
1

<210> 5
<211> 3
<212> PRT
<213> Artificial Sequnece

<220>
<223> Synthetic Peptide

<400> 5
Gly Arg Arg
1

<210> 6
<211> 3
<212> PRT
<213> Artificial Sequnece

<220>
<223> Synthetic Peptide

<400> 6

Asn Gly Lys
1

<210> 7
<211> 3
<212> PRT
<213> Artificial Sequnece

<220>
<223> Synthetic Peptide

<400> 7
Asn Gly Arg
1

<210> 8
<211> 4
<212> PRT
<213> Artificial Sequnece

<220>
<223> Synthetic Peptide

<400> 8
Asn Gly Lys Arg
1

<210> 9
<211> 4
<212> PRT
<213> Artificial Sequnece

<220>
<223> Synthetic Peptide

<400> 9
Asn Gly Arg Arg
1

<210> 10
<211> 11
<212> PRT
<213> Artificial Sequnece

<220>
<223> Synthetic Peptide

<220>
<221> PEPTIDE
<222> 2
<223> Xaa represents Lys or Arg

<220>
<221> PEPTIDE
<222> 3
<223> Xaa represents Lys or Arg

<220>
<221> PEPTIDE
<222> 5
<223> Xaa represents Lys or Arg

<220>
<221> PEPTIDE
<222> 7
<223> Xaa represents Lys or Arg

<220>
<221> PEPTIDE
<222> 9
<223> Xaa represents Lys or Arg

<220>
<221> PEPTIDE
<222> 11
<223> Xaa represents Lys or Arg

<400> 10
Gly Xaa Xaa Tyr Xaa Gln Xaa Val Xaa Asn Xaa
1               5                   10

<210> 11
<211> 12
<212> PRT
<213> Artificial Sequnece

<220>
<223> Synthetic Peptide

<220>
<221> PEPTIDE
<222> 3
<223> Xaa represents Lys or Arg

<220>
<221> PEPTIDE
<222> 4
<223> Xaa represents Lys or Arg

<220>
<221> PEPTIDE
<222> 6
<223> Xaa represents Lys or Arg

<220>
<221> PEPTIDE
<222> 8
<223> Xaa represents Lys or Arg

<220>
<221> PEPTIDE
<222> 10
<223> Xaa represents Lys or Arg

<220>
<221> PEPTIDE
<222> 12
<223> Xaa represents Lys or Arg

<400> 11
Asn Gly Xaa Xaa Tyr Xaa Gln Xaa Val Xaa Asn Xaa
1               5                   10

<210> 12
<211> 38
<212> PRT
<213> Artificial Sequence

<220>
<223> Synthetic Peptide

<400> 12
His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu Gly Lys Arg Tyr Lys
            20                  25                  30

Gln Arg Val Lys Asn Lys
        35

<210> 13
<211> 27
<212> PRT
<213> Artificial Sequence

<220>
<223> Synthetic Peptide

<400> 13
His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu
            20                  25

<210> 14
<211> 28
<212> PRT
<213> Artificial Sequence

<220>
<223> Synthetic Peptide

<400> 14
His Ser Asp Ala Val Phe Thr Asp Asn Tyr Thr Arg Leu Arg Lys Gln
1               5                   10                  15

Met Ala Val Lys Lys Tyr Leu Asn Ser Ile Leu Asn
            20                  25

<210> 15
<211> 38
<212> PRT
<213> Artificial Sequence

<220>
<223> Synthetic Peptide

<400> 15
His Ser Asp Gly Ile Phe Thr Asp Ser Tyr Ser Arg Tyr Arg Arg Gln

1                   5                   10                  15

Leu Ala Val Arg Arg Tyr Leu Ala Ala Val Leu Gly Arg Arg Tyr Arg
                20                  25                  30

Gln Arg Val Arg Asn Arg
            35



<210> 16
<211> 38
<212> PRT
<213> Artificial Sequence

<220>
<223> Synthetic Peptide

<400> 16
His Ser Asp Gly Ile Phe Thr Asp Ser Tyr Ser Arg Tyr Arg Lys Gln
1                   5                   10                  15

Met Ala Val Gln Lys Tyr Leu Ala Ala Val Leu Gly Arg Arg Tyr Arg
                20                  25                  30

Gln Arg Val Arg Asn Lys
            35



<210> 17
<211> 27
<212> PRT
<213> Artificial Sequence

<220>
<223> Synthetic Peptide

<400> 17
His Ser Asp Gly Ile Phe Thr Asp Ser Tyr Ser Arg Tyr Arg Arg Gln

1               5                   10                  15

Leu Ala Val Arg Arg Tyr Leu Ala Ala Val Leu
            20                  25

<210> 18
<211> 27
<212> PRT
<213> Artificial Sequence

<220>
<223> Synthetic Peptide

<400> 18
His Ser Asp Ala Val Phe Thr Asp Ser Tyr Ser Arg Tyr Arg Lys Gln
1               5                   10                  15

Met Ala Val Lys Lys Tyr Leu Ala Ala Val Leu
            20                  25

<210> 19
<211> 27
<212> PRT
<213> Artificial Sequence

<220>
<223> Synthetic Peptide

<400> 19
His Ser Asp Ala Val Phe Thr Asp Asn Tyr Ser Arg Tyr Arg Lys Gln
1               5                   10                  15

Met Ala Val Lys Lys Tyr Leu Ala Ala Val Leu
            20                  25

<210> 20
<211> 28
<212> PRT
<213> Artificial Sequence

<220>
<223> Synthetic Peptide

<400> 20
His Ser Asp Gly Ile Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 21
<211> 28
<212> PRT
<213> Artificial Sequence

<220>
<223> Synthetic Peptide

<400> 21
His Ser Asp Gly Ile Phe Thr Asp Ser Tyr Thr Arg Leu Arg Lys Gln
1               5                   10                  15

Met Ala Val Lys Lys Tyr Leu Asn Ser Ile Leu Asn
            20                  25


<210> 22
<211> 30
<212> PRT
<213> Artificial Sequence

<220>

<223> Synthetic Peptide

<400> 22
His Ser Asp Ala Val Phe Thr Asp Asn Tyr Thr Arg Leu Arg Arg Gln
1               5                   10                  15

Leu Ala Val Arg Arg Tyr Leu Asn Ser Ile Leu Asn Gly Lys
            20                  25                  30


<210> 23
<211> 31
<212> PRT
<213> Artificial Sequence

<220>
<223> Synthetic Peptide

<400> 23
His Ser Asp Ala Val Phe Thr Asp Asn Tyr Thr Arg Leu Arg Arg Gln
1               5                   10                  15

Leu Ala Val Arg Arg Tyr Leu Asn Ser Ile Leu Asn Gly Lys Arg
            20                  25                  30


<210> 24
<211> 31
<212> PRT
<213> Artificial Sequence

<220>
<223> Synthetic Peptide

<400> 24
His Ser Asp Ala Val Phe Thr Asp Asn Tyr Thr Arg Leu Arg Arg Gln
1               5                   10                  15

Leu Ala Val Arg Arg Tyr Leu Asn Ser Ile Leu Asn Gly Arg Arg
            20                  25                  30

<210> 25
<211> 30
<212> PRT
<213> Artificial Sequence

<220>
<223> Synthetic Peptide

<400> 25
His Ser Asp Ala Val Phe Thr Asp Asn Tyr Thr Arg Leu Arg Arg Gln
 1               5                  10                  15

Ala Val Arg Arg Tyr Leu Asn Ser Ile Leu Asn Gly Arg Arg
            20                  25                  30

<210> 26
<211> 29
<212> PRT
<213> Artificial Sequence

<220>
<223> Synthetic Peptide

<400> 26
His Ser Asp Gly Ile Phe Thr Asp Ser Tyr Ser Arg Tyr Arg Arg Gln
 1               5                  10                  15

Leu Ala Val Arg Arg Tyr Leu Ala Ala Val Leu Gly Lys
            20                  25

<210> 27
<211> 30

<212> PRT
<213> Artificial Sequence

<220>
<223> Synthetic Peptide

<400> 27
His Ser Asp Gly Ile Phe Thr Asp Ser Tyr Ser Arg Tyr Arg Arg Gln
1               5                   10                  15

Leu Ala Val Arg Arg Tyr Leu Ala Ala Val Leu Gly Lys Arg
            20                  25                  30


<210> 28
<211> 30
<212> PRT
<213> Artificial Sequence

<220>
<223> Synthetic Peptide

<400> 28
His Ser Asp Gly Ile Phe Thr Asp Ser Tyr Ser Arg Tyr Arg Arg Gln
1               5                   10                  15

Leu Ala Val Arg Arg Tyr Leu Ala Ala Val Leu Gly Arg Arg
            20                  25                  30


<210> 29
<211> 38
<212> PRT
<213> Artificial Sequence

<220>
<223> Synthetic Peptide

<400> 29
His Ser Asp Ala Val Phe Thr Asp Asn Tyr Thr Arg Leu Arg Arg Gln
1               5                   10                  15

Ala Val Arg Arg Tyr Leu Asn Ser Ile Leu Asn Gly Arg Arg Tyr Arg
            20                  25                  30

Gln Arg Val Arg Asn Arg
        35

<210> 30
<211> 23
<212> PRT
<213> Artificial Sequence

<220>
<223> Synthetic Peptide

<400> 30
His Ser Asp Gly Ile Phe Thr Asp Ser Tyr Ser Arg Tyr Arg Lys Gln
1               5                   10                  15

Met Ala Val Lys Lys Tyr Leu
            20

<210> 31
<211> 23
<212> PRT
<213> Artificial Sequence

<220>
<223> Synthetic Peptide

<400> 31
His Ser Asp Ala Val Phe Thr Asp Asn Tyr Thr Arg Leu Arg Lys Gln
1               5                   10                  15

Met Ala Val Lys Lys Tyr Leu
            20

<210> 32
<211> 44
<212> PRT
<213> Artificial Sequence

<220>
<223> Synthetic Peptide

<400> 32
His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu Gly Lys Arg Tyr Lys
            20                  25                  30

Pro Lys Arg Arg Asn Ser Gly Arg Arg Val Phe Tyr
        35                  40

<210> 33
<211> 24
<212> PRT
<213> Artificial Sequence

<220>
<223> Synthetic Peptide

<400> 33
His Ser Asp Ala Val Phe Thr Asp Asn Tyr Thr Arg Leu Arg Lys Gln
1               5                   10                  15

Met Ala Val Lys Lys Tyr Leu Asn
            20

<210> 34
<211> 25
<212> PRT
<213> Artificial Sequence

<220>
<223> Synthetic Peptide

<400> 34
His Ser Asp Ala Val Phe Thr Asp Asn Tyr Thr Arg Leu Arg Lys Gln
1               5                   10                  15

Met Ala Val Lys Lys Tyr Leu Asn Ser
            20                  25


<210> 35
<211> 26
<212> PRT
<213> Artificial Sequence

<220>
<223> Synthetic Peptide

<400> 35
His Ser Asp Ala Val Phe Thr Asp Asn Tyr Thr Arg Leu Arg Lys Gln
1               5                   10                  15

Met Ala Val Lys Lys Tyr Leu Asn Ser Ile
            20                  25


<210> 36
<211> 27
<212> PRT
<213> Artificial Sequence

<220>
<223> Synthetic Peptide

<400> 36
His Ser Asp Ala Val Phe Thr Asp Asn Tyr Thr Arg Leu Arg Lys Gln
1               5                   10                  15

Met Ala Val Lys Lys Tyr Leu Asn Ser Ile Leu
            20                  25


<210> 37
<211> 24
<212> PRT
<213> Artificial Sequence

<220>
<223> Synthetic Peptide

<400> 37
His Ser Asp Gly Ile Phe Thr Asp Ser Tyr Ser Arg Tyr Arg Lys Gln
1               5                   10                  15

Met Ala Val Lys Lys Tyr Leu Ala
            20


<210> 38
<211> 25
<212> PRT
<213> Artificial Sequence

<220>
<223> Synthetic Peptide

<400> 38
His Ser Asp Gly Ile Phe Thr Asp Ser Tyr Ser Arg Tyr Arg Lys Gln
1               5                   10                  15

Met Ala Val Lys Lys Tyr Leu Ala Ala
            20                  25

<210> 39
<211> 26
<212> PRT
<213> Artificial Sequence

<220>
<223> Synthetic Peptide

<400> 39
His Ser Asp Gly Ile Phe Thr Asp Ser Tyr Ser Arg Tyr Arg Lys Gln
 1               5                  10                  15

Met Ala Val Lys Lys Tyr Leu Ala Ala Val
            20                  25

<210> 40
<211> 28
<212> PRT
<213> Artificial Sequence

<220>
<223> Synthetic Peptide

<400> 40
His Ser Asp Gly Ile Phe Thr Asp Ser Tyr Ser Arg Tyr Arg Lys Gln
 1               5                  10                  15

Met Ala Val Lys Lys Tyr Leu Ala Ala Val Leu Gly
            20                  25

<210> 41

<211> 29
<212> PRT
<213> Artificial Sequence

<220>
<223> Synthetic Peptide

<400> 41
His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu Gly Lys
            20                  25


<210> 42
<211> 30
<212> PRT
<213> Artificial Sequence

<220>
<223> Synthetic Peptide

<400> 42
His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu Gly Lys Arg
            20                  25                  30


<210> 43
<211> 31
<212> PRT
<213> Artificial Sequence

<220>
<223> Synthetic Peptide

<400> 43
His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu Gly Lys Arg Tyr
            20                  25                  30

<210> 44
<211> 32
<212> PRT
<213> Artificial Sequence

<220>
<223> Synthetic Peptide

<400> 44
His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu Gly Lys Arg Tyr Lys
            20                  25                  30

<210> 45
<211> 33
<212> PRT
<213> Artificial Sequence

<220>
<223> Synthetic Peptide

<400> 45
His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu Gly Lys Arg Tyr Lys

20                    25                    30

Gln

<210> 46
<211> 34
<212> PRT
<213> Artificial Sequence

<220>
<223> Synthetic Peptide

<400> 46
His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu Gly Lys Arg Tyr Lys
            20                  25                  30

Gln Arg

<210> 47
<211> 35
<212> PRT
<213> Artificial Sequence

<220>
<223> Synthetic Peptide

<400> 47
His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu Gly Lys Arg Tyr Lys

20                  25                  30

Gln Arg Val
        35

<210> 48
<211> 36
<212> PRT
<213> Artificial Sequence

<220>
<223> Synthetic Peptide

<400> 48
His Ser Asp Gly Ile Phe Thr Asp Ser Tyr Ser Arg Tyr Arg Lys Gln
  1               5                  10                  15

Met Ala Val Lys Lys Tyr Leu Ala Ala Val Leu Gly Lys Arg Tyr Lys
              20                  25                  30

Gln Arg Val Lys
        35

<210> 49
<211> 37
<212> PRT
<213> Artificial Sequence

<220>
<223> Synthetic Peptide

<400> 49
His Ser Asp Gly Ile Phe Thr Asp Ser Tyr Ser Arg Tyr Arg Lys Gln
  1               5                  10                  15

Met Ala Val Lys Lys Tyr Leu Ala Ala Val Leu Gly Lys Arg Tyr Lys

20                    25                    30

Gln Arg Val Lys Asn
        35

SEQUENCE LISTING

<110> ITOHAM FOODS INC.

<120> REMEDIES AND/OR PREVENTATIVES FOR CONFORMATIONAL DISEASES

<130> N.89606 GCW

<140> 02805029.2
<141> 2002-12-19

<150> PCT/JP02/13311
<151> 2002-12-19

<150> JP 2001-386699
<151> 2001-12-19

<160> 49

<170> PatentIn Ver. 2.1

<210> 1
<211> 27
<212> PRT
<213> Artificial Sequence

<220>
<223> Synthetic Peptide

<220>
<221> PEPTIDE
<222> 4
<223> Xaa represents Ala or Gly

<220>
<221> PEPTIDE
<222> 5
<223> Xaa represents Ile or Val

<220>
<221> PEPTIDE
<222> 9
<223> Xaa represents Asn or Ser

<220>
<221> PEPTIDE
<222> 11
<223> Xaa represents Thr or Ser

<220>
<221> PEPTIDE
<222> 13
<223> Xaa represents Leu or Tyr

<220>
<221> PEPTIDE
<222> 15
<223> Xaa represents Lys or Arg

<220>
<221> PEPTIDE
<222> 17
<223> Xaa represents Met or Leu or nLeu

<220>
<221> PEPTIDE
<222> 20
<223> Xaa represents Lys or Arg or Gln

<220>
<221> PEPTIDE
<222> 21
<223> Xaa represents Lys or Arg

<220>
<221> PEPTIDE
<222> 24
<223> Xaa represents Asn or Ala

<220>
<221> PEPTIDE
<222> 25
<223> Xaa represents Ser or Ala

<220>
<221> PEPTIDE
<222> 26
<223> Xaa represents Ile or Val

<400> 1
His Ser Asp Xaa Xaa Phe Thr Asp Xaa Tyr Xaa Arg Xaa Arg Xaa Gln
1               5                   10                  15
Xaa Ala Val Xaa Xaa Tyr Leu Xaa Xaa Xaa Leu
            20                  25

<210> 2
<211> 2

<212> PRT
<213> Artificial Sequence

<220>
<223> Synthetic Peptide

<400> 2
Gly Lys
1


<210> 3
<211> 2
<212> PRT
<213> Artificial Sequence

<220>
<223> Synthetic Peptide

<400> 3
Gly Arg
1


<210> 4
<211> 3
<212> PRT
<213> Artificial Sequence

<220>
<223> Synthetic Peptide

<400> 4
Gly Lys Arg
1


<210> 5
<211> 3
<212> PRT
<213> Artificial Sequence

<220>
<223> Synthetic Peptide

<400> 5
Gly Arg Arg
1

<210> 6
<211> 3
<212> PRT
<213> Artificial Sequence

<220>
<223> Synthetic Peptide

<400> 6
Asn Gly Lys
1

<210> 7
<211> 3
<212> PRT
<213> Artificial Sequence

<220>
<223> Synthetic Peptide

<400> 7
Asn Gly Arg
1

<210> 8
<211> 4
<212> PRT
<213> Artificial Sequence

<220>
<223> Synthetic Peptide

<400> 8
Asn Gly Lys Arg
1

<210> 9
<211> 4
<212> PRT
<213> Artificial Sequence

<220>
<223> Synthetic Peptide

<400> 9

Asn Gly Arg Arg
1

<210> 10
<211> 11
<212> PRT
<213> Artificial Sequence

<220>
<223> Synthetic Peptide

<220>
<221> PEPTIDE
<222> 2
<223> Xaa represents Lys or Arg

<220>
<221> PEPTIDE
<222> 3
<223> Xaa represents Lys or Arg

<220>
<221> PEPTIDE
<222> 5
<223> Xaa represents Lys or Arg

<220>
<221> PEPTIDE
<222> 7
<223> Xaa represents Lys or Arg

<220>
<221> PEPTIDE
<222> 9
<223> Xaa represents Lys or Arg

<220>
<221> PEPTIDE
<222> 11
<223> Xaa represents Lys or Arg

<400> 10
Gly Xaa Xaa Tyr Xaa Gln Xaa Val Xaa Asn Xaa
1               5                   10

<210> 11
<211> 12

<212> PRT
<213> Artificial Sequence

<220>
<223> Synthetic Peptide

<220>
<221> PEPTIDE
<222> 3
<223> Xaa represents Lys or Arg

<220>
<221> PEPTIDE
<222> 4
<223> Xaa represents Lys or Arg

<220>
<221> PEPTIDE
<222> 6
<223> Xaa represents Lys or Arg

<220>
<221> PEPTIDE
<222> 8
<223> Xaa represents Lys or Arg

<220>
<221> PEPTIDE
<222> 10
<223> Xaa represents Lys or Arg

<220>
<221> PEPTIDE
<222> 12
<223> Xaa represents Lys or Arg

<400> 11
Asn Gly Xaa Xaa Tyr Xaa Gln Xaa Val Xaa Asn Xaa
1               5                   10


<210> 12
<211> 38
<212> PRT
<213> Artificial Sequence

<220>
<223> Synthetic Peptide

<400> 12
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu Gly Lys Arg Tyr Lys
            20                  25                  30
Gln Arg Val Lys Asn Lys
        35

<210> 13
<211> 27
<212> PRT
<213> Artificial Sequence

<220>
<223> Synthetic Peptide

<400> 13
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu
            20                  25

<210> 14
<211> 28
<212> PRT
<213> Artificial Sequence

<220>
<223> Synthetic Peptide

<400> 14
His Ser Asp Ala Val Phe Thr Asp Asn Tyr Thr Arg Leu Arg Lys Gln
1               5                   10                  15
Met Ala Val Lys Lys Tyr Leu Asn Ser Ile Leu Asn
            20                  25

<210> 15
<211> 38
<212> PRT
<213> Artificial Sequence

<220>
<223> Synthetic Peptide

<400> 15

His Ser Asp Gly Ile Phe Thr Asp Ser Tyr Ser Arg Tyr Arg Arg Gln
1               5                   10                  15
Leu Ala Val Arg Arg Tyr Leu Ala Ala Val Leu Gly Arg Arg Tyr Arg
            20                  25                  30
Gln Arg Val Arg Asn Arg
        35

<210> 16
<211> 38
<212> PRT
<213> Artificial Sequence

<220>
<223> Synthetic Peptide

<400> 16
His Ser Asp Gly Ile Phe Thr Asp Ser Tyr Ser Arg Tyr Arg Lys Gln
1               5                   10                  15
Met Ala Val Gln Lys Tyr Leu Ala Ala Val Leu Gly Arg Arg Tyr Arg
            20                  25                  30
Gln Arg Val Arg Asn Lys
        35

<210> 17
<211> 27
<212> PRT
<213> Artificial Sequence

<220>
<223> Synthetic Peptide

<400> 17
His Ser Asp Gly Ile Phe Thr Asp Ser Tyr Ser Arg Tyr Arg Arg Gln
1               5                   10                  15
Leu Ala Val Arg Arg Tyr Leu Ala Ala Val Leu
            20                  25

<210> 18
<211> 27
<212> PRT
<213> Artificial Sequence

<220>
<223> Synthetic Peptide

<400> 18

His Ser Asp Ala Val Phe Thr Asp Ser Tyr Ser Arg Tyr Arg Lys Gln
1               5                   10                  15
Met Ala Val Lys Lys Tyr Leu Ala Ala Val Leu
            20                  25

<210> 19
<211> 27
<212> PRT
<213> Artificial Sequence

<220>
<223> Synthetic Peptide

<400> 19
His Ser Asp Ala Val Phe Thr Asp Asn Tyr Ser Arg Tyr Arg Lys Gln
1               5                   10                  15
Met Ala Val Lys Lys Tyr Leu Ala Ala Val Leu
            20                  25

<210> 20
<211> 28
<212> PRT
<213> Artificial Sequence

<220>
<223> Synthetic Peptide

<400> 20
His Ser Asp Gly Ile Phe Thr Asp Asn Tyr Thr Arg Leu Arg Lys Gln
1               5                   10                  15
Met Ala Val Lys Lys Tyr Leu Asn Ser Ile Leu Asn
            20                  25

<210> 21
<211> 28
<212> PRT
<213> Artificial Sequence

<220>
<223> Synthetic Peptide

<400> 21
His Ser Asp Gly Ile Phe Thr Asp Ser Tyr Thr Arg Leu Arg Lys Gln
1               5                   10                  15
Met Ala Val Lys Lys Tyr Leu Asn Ser Ile Leu Asn
            20                  25

<210> 22
<211> 30
<212> PRT
<213> Artificial Sequence

<220>
<223> Synthetic Peptide

<400> 22
His Ser Asp Ala Val Phe Thr Asp Asn Tyr Thr Arg Leu Arg Arg Gln
1               5                   10                  15
Leu Ala Val Arg Arg Tyr Leu Asn Ser Ile Leu Asn Gly Lys
            20                  25                  30


<210> 23
<211> 31
<212> PRT
<213> Artificial Sequence

<220>
<223> Synthetic Peptide

<400> 23
His Ser Asp Ala Val Phe Thr Asp Asn Tyr Thr Arg Leu Arg Arg Gln
1               5                   10                  15
Leu Ala Val Arg Arg Tyr Leu Asn Ser Ile Leu Asn Gly Lys Arg
            20                  25                  30


<210> 24
<211> 31
<212> PRT
<213> Artificial Sequence

<220>
<223> Synthetic Peptide

<400> 24
His Ser Asp Ala Val Phe Thr Asp Asn Tyr Thr Arg Leu Arg Arg Gln
1               5                   10                  15
Leu Ala Val Arg Arg Tyr Leu Asn Ser Ile Leu Asn Gly Arg Arg
            20                  25                  30


<210> 25
<211> 30

<212> PRT
<213> Artificial Sequence

<220>
<223> Synthetic Peptide

<400> 25
His Ser Asp Ala Val Phe Thr Asp Asn Tyr Thr Arg Leu Arg Arg Gln
1               5                   10                  15
Ala Val Arg Arg Tyr Leu Asn Ser Ile Leu Asn Gly Arg Arg
            20                  25                  30


<210> 26
<211> 29
<212> PRT
<213> Artificial Sequence

<220>
<223> Synthetic Peptide

<400> 26
His Ser Asp Gly Ile Phe Thr Asp Ser Tyr Ser Arg Tyr Arg Arg Gln
1               5                   10                  15
Leu Ala Val Arg Arg Tyr Leu Ala Ala Val Leu Gly Lys
            20                  25


<210> 27
<211> 30
<212> PRT
<213> Artificial Sequence

<220>
<223> Synthetic Peptide

<400> 27
His Ser Asp Gly Ile Phe Thr Asp Ser Tyr Ser Arg Tyr Arg Arg Gln
1               5                   10                  15
Leu Ala Val Arg Arg Tyr Leu Ala Ala Val Leu Gly Lys Arg
            20                  25                  30


<210> 28
<211> 30
<212> PRT
<213> Artificial Sequence

<220>

<223> Synthetic Peptide

<400> 28
His Ser Asp Gly Ile Phe Thr Asp Ser Tyr Ser Arg Tyr Arg Arg Gln
1               5                   10                  15
Leu Ala Val Arg Arg Tyr Leu Ala Ala Val Leu Gly Arg Arg
            20                  25                  30

<210> 29
<211> 38
<212> PRT
<213> Artificial Sequence

<220>
<223> Synthetic Peptide

<400> 29
His Ser Asp Ala Val Phe Thr Asp Asn Tyr Thr Arg Leu Arg Arg Gln
1               5                   10                  15
Ala Val Arg Arg Tyr Leu Asn Ser Ile Leu Asn Gly Arg Arg Tyr Arg
            20                  25                  30
Gln Arg Val Arg Asn Arg
        35

<210> 30
<211> 23
<212> PRT
<213> Artificial Sequence

<220>
<223> Synthetic Peptide

<400> 30
His Ser Asp Gly Ile Phe Thr Asp Ser Tyr Ser Arg Tyr Arg Lys Gln
1               5                   10                  15
Met Ala Val Lys Lys Tyr Leu
            20

<210> 31
<211> 23
<212> PRT
<213> Artificial Sequence

<220>
<223> Synthetic Peptide

<400> 31
His Ser Asp Ala Val Phe Thr Asp Asn Tyr Thr Arg Leu Arg Lys Gln
1               5                   10                  15
Met Ala Val Lys Lys Tyr Leu
            20

<210> 32
<211> 44
<212> PRT
<213> Artificial Sequence

<220>
<223> Synthetic Peptide

<400> 32
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu Gly Lys Arg Tyr Lys
            20                  25                  30
Pro Lys Arg Arg Asn Ser Gly Arg Arg Val Phe Tyr
        35                  40

<210> 33
<211> 24
<212> PRT
<213> Artificial Sequence

<220>
<223> Synthetic Peptide

<400> 33
His Ser Asp Ala Val Phe Thr Asp Asn Tyr Thr Arg Leu Arg Lys Gln
1               5                   10                  15
Met Ala Val Lys Lys Tyr Leu Asn
            20

<210> 34
<211> 25
<212> PRT
<213> Artificial Sequence

<220>
<223> Synthetic Peptide

<400> 34
His Ser Asp Ala Val Phe Thr Asp Asn Tyr Thr Arg Leu Arg Lys Gln
1               5                   10                  15

Met Ala Val Lys Lys Tyr Leu Asn Ser
            20                  25

<210> 35
<211> 26
<212> PRT
<213> Artificial Sequence

<220>
<223> Synthetic Peptide

<400> 35
His Ser Asp Ala Val Phe Thr Asp Asn Tyr Thr Arg Leu Arg Lys Gln
1               5                   10                  15
Met Ala Val Lys Lys Tyr Leu Asn Ser Ile
            20                  25

<210> 36
<211> 27
<212> PRT
<213> Artificial Sequence

<220>
<223> Synthetic Peptide

<400> 36
His Ser Asp Ala Val Phe Thr Asp Asn Tyr Thr Arg Leu Arg Lys Gln
1               5                   10                  15
Met Ala Val Lys Lys Tyr Leu Asn Ser Ile Leu
            20                  25

<210> 37
<211> 24
<212> PRT
<213> Artificial Sequence

<220>
<223> Synthetic Peptide

<400> 37
His Ser Asp Gly Ile Phe Thr Asp Ser Tyr Ser Arg Tyr Arg Lys Gln
1               5                   10                  15
Met Ala Val Lys Lys Tyr Leu Ala
            20

<210> 38
<211> 25
<212> PRT
<213> Artificial Sequence

<220>
<223> Synthetic Peptide

<400> 38
His Ser Asp Gly Ile Phe Thr Asp Ser Tyr Ser Arg Tyr Arg Lys Gln
1               5                   10                  15
Met Ala Val Lys Lys Tyr Leu Ala Ala
            20                  25


<210> 39
<211> 26
<212> PRT
<213> Artificial Sequence

<220>
<223> Synthetic Peptide

<400> 39
His Ser Asp Gly Ile Phe Thr Asp Ser Tyr Ser Arg Tyr Arg Lys Gln
1               5                   10                  15
Met Ala Val Lys Lys Tyr Leu Ala Ala Val
            20                  25


<210> 40
<211> 28
<212> PRT
<213> Artificial Sequence

<220>
<223> Synthetic Peptide

<400> 40
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu Gly
            20                  25


<210> 41
<211> 29
<212> PRT
<213> Artificial Sequence

<220>
<223> Synthetic Peptide

<400> 41
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu Gly Lys
            20                  25


<210> 42
<211> 30
<212> PRT
<213> Artificial Sequence

<220>
<223> Synthetic Peptide

<400> 42
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu Gly Lys Arg
            20                  25                  30


<210> 43
<211> 31
<212> PRT
<213> Artificial Sequence

<220>
<223> Synthetic Peptide

<400> 43
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu Gly Lys Arg Tyr
            20                  25                  30


<210> 44
<211> 32
<212> PRT
<213> Artificial Sequence

<220>
<223> Synthetic Peptide

<400> 44
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu Gly Lys Arg Tyr Lys
            20                  25                  30

<210> 45
<211> 33
<212> PRT
<213> Artificial Sequence

<220>
<223> Synthetic Peptide

<400> 45
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu Gly Lys Arg Tyr Lys
            20                  25                  30
Gln

<210> 46
<211> 34
<212> PRT
<213> Artificial Sequence

<220>
<223> Synthetic Peptide

<400> 46
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu Gly Lys Arg Tyr Lys
            20                  25                  30
Gln Arg

<210> 47
<211> 35
<212> PRT
<213> Artificial Sequence

<220>
<223> Synthetic Peptide

<400> 47
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu Gly Lys Arg Tyr Lys
            20                  25                  30
Gln Arg Val
        35

<210> 48
<211> 36
<212> PRT
<213> Artificial Sequence

<220>
<223> Synthetic Peptide

<400> 48
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu Gly Lys Arg Tyr Lys
            20                  25                  30
Gln Arg Val Lys
        35

<210> 49
<211> 37
<212> PRT
<213> Artificial Sequence

<220>
<223> Synthetic Peptide

<400> 49
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu Gly Lys Arg Tyr Lys
            20                  25                  30
Gln Arg Val Lys Asn
        35